# EUROPÄISCHE PATENTSCHRIFT

(11) **EP 0 472 501 B1**
(45) Veröffentlichungstag und Bekanntmachung des Hinweises auf die Patenterteilung: **08.05.1996**
(21) Anmeldenummer: 91810645.1
(22) Anmeldetag: 14.08.1991
(51) Int. Cl.: C07D 489/04, A61K 31/645

(54) **Codeinsalz einer substituierten Carbonsäure, Verfahren zu seiner Herstellung, seine Verwendung und pharmazeutische Präparate**
Codeinsalt of a substituted carbonacid, process of its preparation, its use and pharmaceutical preparations
Sel de codéine et d'un acide carbonique substitué, procédé pour sa préparation, son utilisation et préparations pharmaceutiques

(30) Priorität: 23.08.1990 CH 2735/90
(43) Veröffentlichungstag der Anmeldung: 26.02.1992
(73) Patentinhaber: CIBA-GEIGY AG, CH-4002 Basel (CH)
(72) Erfinder: Riess, Walter, Dr., CH-4052 Basel (CH); Sallmann, Afred, Dr., CH-4103 Bottmingen (CH)

(56) Entgegenhaltungen:
- FR-A- 2 100 936
- FR-A- 2 500 751
- GB-A- 2 170 709
- US-A- 4 176 186

## Beschreibung

Die vorliegende Erfindung betrifft ein neues Codeinsalz einer substituierten Carbonsäure, das sich aus zwei verschiedenen Komponenten mit zentralen bzw. peripheren Wirkungseigenschaften zusammensetzt, wobei eine Steigerung der erwünschten analgetischen Wirksamkeit bei gleichzeitiger Geschmacksverbesserung auftritt.

Eines der lang existierenden primären Ziele der Medizin ist die Schmerzlinderung, insbesondere Verbesserung dieser. Diese wird im allgemeinen durch die Verabreichung von zentral oder peripher analgetisch wirksamen Arzneimitteln erzielt, die, einzeln oder in Kombination verabreicht, einen Anstieg des Schmerzschwellenwertes hervorrufen. Es ist schwierig, diesen Anforderungen in den meisten Fällen mit einer einzigen chemischen Komponente gerecht zu werden, da im allgemeinen ein starkes zentrales Analgetikum beträchtliche begleitende Nebenreaktionen hervorruft. Periphere Analgetika haben andere unerwünschte Reaktionen.

Solche unerwünschten Reaktionen können gegebenenfalls gastrointestinale Störungen, Schwindel, Verstopfungen, Uebelkeit und Erbrechen sein. Daher werden beim Einsatz von Analgetika bei Menschen nicht nur der Primäreffekt (Analgesie) berücksichtigt, sondern es wird nach neuen Schmerzmitteln gesucht, die bei maximalen analgetischem Effekt minimale Nebenreaktionen aufweisen. Es besteht daher eine ständige Nachfrage nach einer Wirkungsverbesserung, sei es durch eine Kombination oder andere Massnahmen zweier gleich gerichteter Wirkstoffe, beispielsweise in einer gemeinsamen Salzbildung von kationischen und anionischen Wirkstoffen unter Erhaltung einer einzigen homogenen Substanz gleicher physikalischer Beschaffenheit, wobei die Gesamtmenge der Wirkstoffe reduziert werden kann, die in solchen Mengen eingesetzt werden können, dass sie bei einer maximalen analgetischen Wirkung keine oder nur geringere Nebenwirkungen zeigen. Im allgemeinen wird eine Potenzierung der therapeutischen, d.h. analgetischen Wirkung einerseits und andererseits eine Verminderung der unerwünschten Nebenwirkungen gewünscht.

Aus der DE-A-3 603 564 ist ein pharmazeutisches Präparat mit starker analgetischer Wirkung bekannt, welches sich aus dem Salz von Diclofenac, insbesondere dem Natriumsalz und einem Salz des Codein, insbesondere Codeinphosphat zusammensetzt. Es wurde gefunden, dass eine solche gewünschte potenzierende Wirkung bei einer Kombination von einem pharmazeutisch annehmbaren Salz von Diclofenac mit einem pharmazeutisch annehmbaren Salz von Codein auftritt. Nachteilig bei der oralen Verabreichung der festen Kombination mit einer 1:1 Mischung von Diclofenac-Natrium und Codeinphosphat kann der anhaltend äusserst bittere und in der Gaumen- und Rachengegend sogar ätzende Geschmack bezeichnet werden, der auch des öfteren zum Husten reizt. Insbesondere ist diese Reizwirkung und der damit verbundene Husten von besonderem Nachteil für Krebspatienten, die diese Kombination bestehend aus Diclofenac-Natrium und Codeinphosphat zur Bekämpfung der auftretenden Schmerzen als starkes Analgetikum verabreicht bekommen.

Gegenstand der Erfindung ist ein neues Codeinsalz der [2-[(2,6-Dichlorphenyl)-amino]-phenyl]-essigsäure (Diclofenac) der Formel 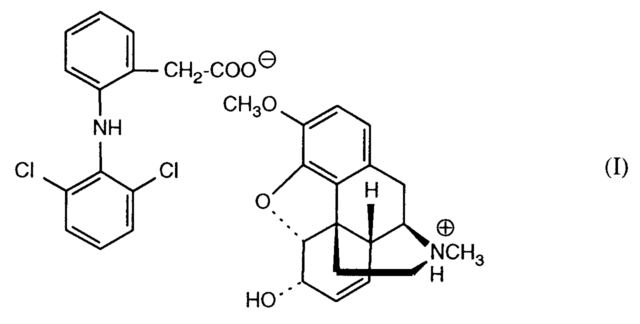
Verfahren zu seiner Herstellung, seine Verwendung als Arzneimittel, sowie pharmazeutische Präparate, die dieses Salz enthalten.

Ueberraschenderweise konnte gefunden werden, dass das erfindungsgemässe Codeinsalz der Formel I mit starker analgetischer Wirkung als homogene einheitliche Substanz frei vom bitteren Beigeschmack und der daraus herleitbaren Reizwirkung (Husten) ist. Bei 4 Testpersonen haben Geschmacksvergleiche ergeben, wobei 2 mg Testsubstanz auf die Zunge gegeben und erst nach 30 Sekunden geschluckt werden, dass das Codeinsalz von Diclofenac der Formel I bei 2 Testpersonen keinen bitteren Geschmack auf der Zunge und bei 2 Testpersonen nur einen schwach bitteren Geschmack hervorruft, wogegen von der kombinierten 1:1 Mischung von Diclofenac-Natrium und Codeinphosphat bei allen 4 Testpersonen ein sehr bitterer und ätzender Geschmack bis in den Gaumen mit Reizwirkung (Husten) hervorgerufen wird.

Als bemerkenswert kann ferner noch der Vergleich der nach dem Benzochinon-Writhing-Test (combined automated writhing/motility test for testing analgesics, gemäss A. Schweizer, R. Brom und H. Scherrer Agents and Actions Vol 23, 1/2, 29-31, 1988) ermittelten ED₅₀ Werte, welche für das neue Codeinsalz der Formel I einen Wert von 4 mg/kg p.o. im Vergleich zur 1:1 Mischung von Diclofenac-Natrium und Codeinphosphat von 6 mg/kg p.o. ergeben und damit die unterschiedlichen Werte zu Gunsten des Diclofenac-Codein-Salzes als einheitliche Substanz sprechen.

Die dem Codeinsalz der Formel I zugrundeliegende [2-[(2,6-Dichlorphenyl)-amino]-phenyl]-essigsäure sowie deren Salze sind aus der DE-A-1 815 802 und insbesondere deren Ammoniumsalze aus der DE-A-2 935 776 und FR-A-2 500 751 bekannt, ebenso deren anti-inflammatorischen und analgetischen Wirkungen.

Das Natriumsalz (Diclofenac-Natrium) wird beispielsweise als nicht-steroidales Antiinflammatorikum bei der Behandlung entzündlicher Prozesse eingesetzt. Dabei werden die entsprechenden Präparate überwiegend oral, ferner jedoch auch enteral oder auch parenteral verabreicht.

Die zweite Komponente des Salzes der Formel I das Codein ist ein bekanntes zentral wirksames Analgetikum vom Opiattyp. Hinsichtlich des Abhängigkeitspotentials von Codein kann man der Literatur entnehmen, dass die Gefahr der Induktion einer Abhängigkeit mit den üblichen oralen Codeindosen als Bestandteil des Codeinsalzes der Formel I unter ärztlicher Ueberwachung als gering anzusehen ist.

Gegenstand der Erfindung sind ebenso Verfahren zur Herstellung der neuen Verbindung der Formel I, welche nach an sich bekannten Methoden durchgeführt werden.

Eine bevorzugte Verfahrensvariante ist beispielsweise dadurch gekennzeichnet, dass man [2-[(2,6-Dichlorphenyl)-amino]-phenyl] -essigsäure der Formel II 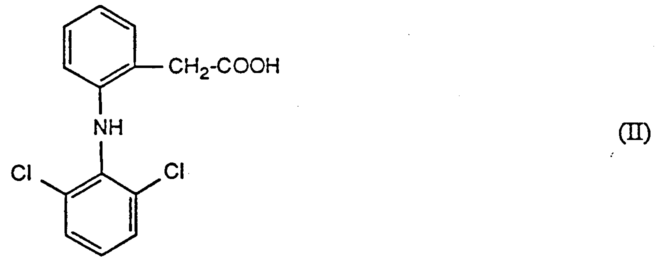
oder ein von dem Salz der Formel I verschiedenes Basesalz davon mit vorzugsweise der mindestens äquimolaren Menge Codein der Formel III 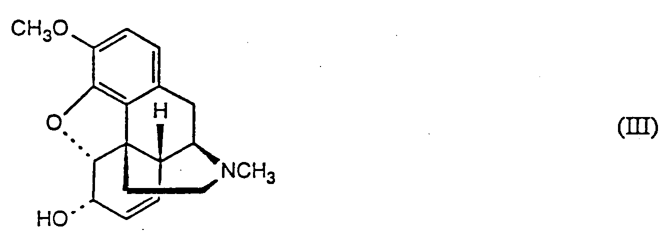
oder einem Salz davon umsetzt.

Verfahrensgemäss verwendbare Salze der Säure der Formel II sind insbesondere Salze mit Basen, die aus dem Reaktionsgemisch entfernt werden können, beispielsweise flüchtiger oder schwächer sind als das Codein der Formel III oder mit [2-[(2,6-Dichlorphenyl)-amino]-phenyl]-essigsäure leichter lösliche Salze bilden als dieses, wie z.B. organische Ammoniumsalze, ebenso Metallsalze, die bei der Umsetzung mit einem geeigneten Salz des Codeins der Formel III schwerlösliche Salze bilden, wie Calciumsalze.

Verfahrensgemäss verwendbare Salze vom Codein der Formel III sind beispielsweise Salze desselben mit Säuren, die aus dem Reaktionsgemisch entfernt werden können, beispeilsweise entsprechende Codeinsalze von flüchtigen Säuren oder von Säuren, die schwächer sauer sind als das entsprechende Salz der Formel I, wie Salze vom Codein der Formel III mit anorganischen Säuren, z.B. Phosphate oder Salze mit organischen Säuren, wie z.B. Fumarate, Maleate oder Oxalate.

Die Reaktion von [2-[(2,6-Dichlorphenyl)-amino]-phenyl]-essigsäure mit Codein erfolgt vorteilhaft in einem inerten Lösungs- bzw. Verdünnungsmittel, erforderlichenfalls unter Kühlen und Erwärmen, z.B. in einem Temperaturbereich von etwa 0° bis etwa 100°C, vorzugsweise bei Raumtemperatur, in einem geschlossenen Gefäss und/oder unter Inertgasatmosphäre, z.B. Stickstoff.

Als geeignetes lösungs- und Verdünnungsmittel kommen beispielsweise Alkohole, wie Niederalkanole, z.B. Methanol oder Aethanol, insbesondere jedoch Aether, wie Diniederalkyläther, z.B. Diäthyläther, sowie cyclische Aether, z.B. Dioxan oder Tetrahydrofuran, Ketone, wie Diniederalkylketone, z.B. Aceton, Carbonsäureester, wie Niederalkancarbonsäureester, z.B. Essigsäureethylester, Amide, wie N,N-Diniederalkyl-amide, z.B. N,N-Dimethylformamid, Sulfoxide, wie Diniederalkylsulfoxide, z.B. Dimethylsulfoxide, oder Gemische derselben in Frage.

[2-[(2,6-Dichlorphenyl)-amino]-phenyl]-essigsäure kann beispielsweise unter den Reaktionsbedingungen aus entsprechenden Estern, wie Niederalkylester, durch Hydrolyse in Gegenwart einer Base, gebildet werden. Codein kann beispielsweise in Form eines Säureadditionssalzes, wie z.B. Phosphates, eingesetzt und gegebenenfalls in Gegenwart einer Base, freigesetzt werden.

In einer bevorzugten Ausführungsform des Verfahrens setzt man beispielsweise die Säure der Formel II in einem organischen Lösungsmittel wie einem Aether, wie Diniederalkyläther, z.B. Diethyläther direkt mit dem Codein der Formel III als Base um. Die Erfindung betrifft auch diejenigen Ausführungsformen des Verfahrens nach denen man die Ausgangsstoffe in situ herstellt, oder bei denen man einen Ausgangsstoff unter den Reaktionsbedingungen aus einem Derivat gewinnt und/oder in Form eines Isomerengemisches oder eines reinen Isomeren einsetzt.

Die Erfindung betrifft weiterhin pharmazeutische Präparate, enthaltend das Salz der Formel I bzw. ein Verfahren zur Herstellung pharmazeutischer Präparate. Das Verfahren ist dadurch gekennzeichnet, dass man die Verbindung der Formel I mit üblichen Hilfs-und/oder Zusatzstoffen vermischt.

Die Erfindung betrifft dabei namentlich die in den Beispielen beschreibenen pharmazeutischen Präparate und Verfahren zu deren Herstellung.

Bei den erfindungsgemässen pharmazeutischen Präparaten, welche Verbindungen der Formel I enthalten, handelt es sich um solche zur enteralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Geeignete Doseneinheitsformen für die orale Verabreichung sind z.B. Tabletten, Manteltabletten (Tabletten mit gepresstem Mantel), Dragées und Kapseln, die 50-300 mg, vorzugsweise 100-250 mg Diclofenac-Codeinsalz, enthalten. Suppositorien und Kapseln für die rektale Verabreichung enthalten 50-200 mg, vorzugsweise 50-150 mg Diclofenac-Codeinsalz. Die Doseneinheitsformen werden ein- bis dreimal täglich in einer Anzahl verabreicht, die einer Tagesdosis von 150-300 mg Diclofenac-Codein für erwachsene Patienten bzw. gemäss Alter und Körpergewicht reduzierten Dosen für Kinder entspricht.

In Dosiseinheitsformen für die perorale Anwendung liegt der Gehalt der beiden als Wirkstoffe verwendeten Verbindungen zusammen vorzugsweise zwischen 20 % und 90 %. Zur Herstellung von Tabletten oder Dragée-Kernen kombiniert man die Wirkstoffe z.B. mit festen, pulverförmigen Trägerstoffen, wie Milchzucker (Lactose), Saccharose, Sorbit, Mannit; Stärken, wie Kartoffelstärke, Maisstärke oder Amylopektin, ferner Laminariapulver oder Citruspulpenpulver; Cellulosederivate, Gelatine oder Polyvinylpyrrolidon, gegebenenfalls unter Zusatz von Gleitmitteln, wie Magnesium- oder Calciumstearat oder Polyäthylenglykolen, wie mit hochdipserser Kieselsäure. Dragée-Kerne überzieht man hierauf beispielsweise mit konz. Zuckerlösungen, welche z.B. noch arabischen Gummi, Talk und/oder Titandioxid enthalten können, oder mit einem in leichtflüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Als orale Doseneinheitsformen eignen sich ferner Steckkapseln aus Gelatine sowie weiche, geschlossene Kapseln aus Gelatine und einem Weichmacher, wie Glycerin. Die ersten enthalten die Wirkstoffe vorzugsweise als Granulat in Mischung mit Gleitmitteln, wie Talk oder Magnesiumstearat, und gegebenenfalls Stäbilisatoren, wie Natriummetabisulfit (Na₂S₂O₅) oder Ascorbinsäure. In weichen Kapseln sind die Wirkstoffe vorzugsweise in geeigneten Flüssigkeiten, wie flüssigen Polyäthylenglykolen, gelöst oder suspendiert, wobei ebenfalls Stabilisatoren zugefügt sein können.

Als Doseneinheitsformen für die rektale Anwendung kommen z.B. Suppositorien in Betracht, welche aus dem kombinierten Wirkstoffe-Salz mit einer Suppositorien-Grundmasse auf der Basis von natürlichen oder synthetischen Triglyceriden von geeignetem Schmelzpunkt (z.B. Kakaobutter), Polyäthylenglykolen oder geeigneten höheren Fettalkoholen bestehen, und Gelatine-Rektalkapseln, welche eine Kombination des Wirkstoffs mit Polyäthylenglykolen enthalten.

Zur parenteralen Verabreichung eignen sich in erster Linie Lösungen des Wirkstoffes, ferner seine Suspensionen, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxy-methylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20 % des Wirkstoffes enthalten.

Creme sind Oel-in-Wasser-Emulsionen, die mehr als 50 % Wasser aufweisen. Als ölige Grundlage verwendet man in erster Linie Fettalkohole, z.B. Lauryl-, Cetyl-oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukten davon, wie Polyglycerinfettsäureester oder Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe etc.

Fettsalben sind wasserfrei und enthalten als Grundlage insbesondere Kohlenwasserstoffe, z.B. Paraffin, Vaseline und/oder flüssige Paraffine, ferner natürliche oder partial-synthetische Fette, z.B. Kokosfettsäuretriglycerid, oder vorzugsweise gehärtete Oele, z.B. hydrierte Erdnuss- oder Rizinusöl, ferner Fettsäurepartialester des Glycerins, z.B. Glycerinmono- oder distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in- Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglykolen, d.h. im wässrigen Gemisch lösliche, lipophile Substanzen als Ersatz für die der Haut mit Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung beigegeben. Die Wirkstoffkonzentration beträgt etwa 0,5-10, z.B. 0,5-5 Gew.%.

Die nachfolgenden Beispiele illustrieren die oben beschriebene Erfindung; sie sollen jedoch diese in ihrem Umfang in keiner Weise einschränken. Temperaturen sind in Celsiusgraden, Drucke in mbar angegeben.

Beispiel 1:
a) 17,84 g Codein werden in einem Rundkolben, der 1300 ml Aether enthält auf dem Wasserbad in Lösung gebracht. Zu dieser Lösung gibt man eine Lösung von 17,65 g [2-[(2,6-Dichlorphenyl)-amino]-phenyl]-essigsäure in 500 ml Aether hinzu und entfernt den Aether als Lösungsmittel teilweise durch Destillation, wobei sich an der Kolbenwand eine harte Kristallkruste bildet. Nach Einengung des Gesamtvolumens an Aether auf ca. 100 ml und Abkühlung des Reaktionsgemisches auf 5°C wird der Kolbeninhalt abgenutscht und das erhaltene Nutschgut mehrmals mit Aether gewaschen, und nach dem Trocknen bei 40°C im Hochvakuum, gesiebt (Sieb 180 µ).
   Man erhält farblose kristalle des Codeinsalzes der [2-[(2,6-Dichlorphenyl)-amino]-phenyl]-essigsäure mit einem Schmelzpunkt von 162-165°C.
b) Die freie Codeinbase wird wie folgt erhalten:
   25,00 g Codeinphosphat werden in 100 ml Eiswasser (Gemisch Eis/Wasser) gelöst und das erhaltene Lösungsgemisch wird mit konzentrierter Natronlauge alkalisch gestellt. Den ausgefallenen harzigen Rückstand nimmt man in Aether auf und gibt nochmals (zweimal) konzentrierte Natronlauge und Wasser hinzu, bis man zwei klare Phasen erhält. Die wässrige Phase wird mehrmals (3x) mit Aether extrahiert und alle gesammelten organischen Phasen über Magnesiumsulfat getroknet und anschliessend im Vakuum eingeengt. Der erhaltene Rückstand wird mit Petroläther versetzt und abgenutscht. Man erhält die freie Codeinbase mit einem Schmelzpunkt von 155-156°C.

Beispiel 2: 3,18 g [2-[(2,6-Dichlorphenyl)-amino]-phenyl]-essigsäure-Natriumsalz werden unter Rühren in 100 ml Wasser bei 65°C gelöst. Die Lösung wird unter Rühren mit einem Eisbad rasch auf 40°C abgekühlt und sofort mit einer Lösung von 4,0 g Codeinphosphat in 50 ml Wasser versetzt. Die entstandene Suspension wird unter Rühren auf 0°C abgekühlt und abfiltriert. Das Nutschgut wird mit 10 ml heissem Wasser verrieben, die Suspension 10 Minuten gerührt und filtriert. Der erhaltene Filterrückstand wird zweimal aus Aethanol umkristallisiert.
Das Codeinsalze der [2-[(2,6-Dichlorphenyl]-amino]-phenyl]-essigsäure schmilzt bei 162-165°C.

Beispiel 3: 3,69 g [2-[(2,6-Dichlorphenyl)-amino]-phenyl]-essigsäure-diäthylammoniumsalz werden unter Rühren in 70 ml Wasser bei 65°C gelöst. Die Lösung wird auf 40°C abgekühlt und mit einer Lösung von 3,97 g Codeinphosphat in 30 ml Wasser versetzt. Die ausgeschiedenen Kristalle werden abfiltriert und mit 10 ml heissem Wasser verrieben. Die Suspension wird nochmals filtriert und der erhaltene feste Rückstand aus Aethanol umkristallisiert.
Das Codeinsalz der [2-[(2,6-Dichlorphenyl)-amino]-phenyl]-essigsäure schmilzt bei 162-165°C.

Beispiel 4: Herstellung von Filmtabletten
400 g Diclofenac-Codeinsalz werden mit 480 g Dicalciumphosphat, 280 g Maisstärke und 48 g kolloidalem Siliciumdioxid gut gemischt. Die Mischung wird mit der Lösung von 64 g Hydroxypropylcellulose (Klucel L®) in 1216 g deionisiertem Wasser in einer dafür geeigneten Vorrichtung sprühgranuliert und getrocknet. Das getrocknete Granulat wird durch ein 1-mm-Sieb passiert. Anschliessend werden zu dem gesiebten Granulat noch 225 g Natriumcarboxymethylstärke (Primojel®), 20 g kolloidales Siliciumdioxid und 8 g Magnesiumstearat dazugemischt. Die fertige Mischung wird in bekannter Weise zu Tabletten mit einem Gewicht von 340 mg verpresst. Die Tabletten besitzen Oblongform und haben eine Bruchkerbe. Anschliessend werden die Tablettenkerne mit einem Ueberzug versehen. Dieser dient dazu, die Tablette leicht schluckbar zu machen. Zu diesem Zweck werden pro Tablette 10 mg Lacksubstanz (nicht magenresistent) in bekannter Weise in einer dazu geeigneten Apparatur aufgetragen. Dazu werden Hydroxypropylmethylcellulose (Pharmacoat®), Polyoxyäthylen-Sorbitan-Fettsäureester (Tween®), Titandioxid und Talkum in deionisiertem Wasser gelöst bzw. suspendiert.

Die überzogene Tablette zerfällt in Wasser bzw. physiologischen Medien rasch und gibt die Wirkstoffe frei.
1 Filmtablette von 350 mg Gewicht enthält:
100 mg Diclofenac-Codeinsalz.

Beispiel 5: Herstellung von Suppositorien
Diclofenac-Codeinsalz, feinst gemahlen, werden in geschmolzener Suppositoriengrundmasse (Hartfett, Ph. Eur. Band III, mit einer Hydroxyzahl von <5) suspendiert. Fettlösliche Farbstoffe (z.B. Chlorophyll) oder Farbpigmente können zugesetzt werden. Die Suspension wird in bekannter Weise in Formen vergossen. Diese Formen sind entweder aus kunststoff vorgeformt und dienen nach dem Verschweissen als Verpackung oder die Formen sind aus Metall. Daraus werden die Suppositorien nach dem Abkühlen herausgelöst und in Folien eingeschweisst. Die Suppositorien sind in der Regel torpedoförmig, weiss oder gefärbt und haben ein Gewicht von ca. 2 g. Nach der rektalen Applikation schmelzen die Suppositorien und geben die inkorporierten Wirkstoffe frei.
1 Suppositorium enthält:
100 mg Diclofenac-Codeinsalz.

## Patentansprüche

1. Das Codeinsalz der [2-[(2,6-Dichlorphenyl)-amino]-phenyl]-essigsäure der Formel

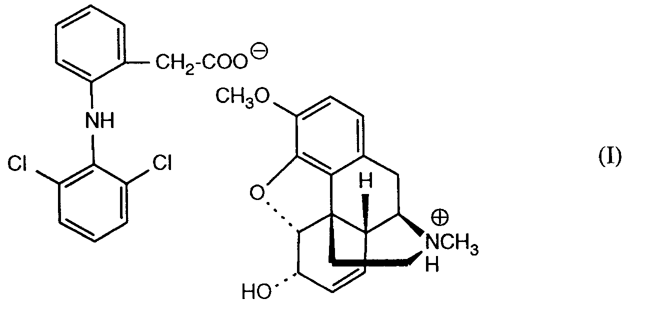

2. Verfahren zur Herstellung des Codeinsalzes der [2-[(2,6-Dichlorphenyl)-amino]phenyl]-essigsäure der Formel I gemäss Anspruch 1, dadurch gekennzeichnet, dass man [2-[(2,6-Dichlorphenyl)-amino]-phenyl]-essigsäure oder ein von dem Salz der Formel I verschiedenes Basesalz davon mit Codein oder einem Salz davon umsetzt.

3. Verfahren gemäss Anspruch 2, dadurch gekennzeichnet, dass man mindestens die äquimolare Menge Codein bzw. einem Salz davon verwendet.

4. Ein pharmazeutisches Präparat enthaltend das Codeinsalz der [2-[(2,6-Dichlorphenyl)-amino]-phenyl]-essigsäure der Formel I gemäss Anspruch 1 neben ublichen pharmazeutischen Hilfsstoffen.

5. Das Codeinsalz der [2-[(2,6-Dichlorphenyl)-amino]-phenyl]-essigsäure der Formel I gemäss Anspruch 1 zur Anwendung in einem Verfahren zur therapeutischen Behandlung des menschlichen oder tierischen Körpers.

6. Verwendung des Codeinsalzes der [2-[(2,6-Dichlorphenyl)-amino]-phenyl]-essigsäure der Formel I gemäss Anspruch 1 für die Herstellung von Medikamenten zur therapeutischen Behandlung des menschlichen oder tierischen Körpers.

## Claims

1. The codeine salt of [2-[(2,6-dichlorophenyl)-amino]phenyl]-acetic acid of formula

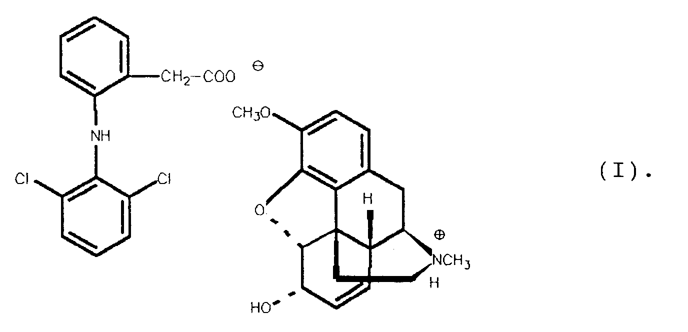

2. A process for the preparation of the codeine salt of [2-[(2,6-dichlorophenyl)-amino]-phenyl]-acetic acid of formula I according to claim 1, which comprises reacting [2-[(2,6-dichlorophenyl)-amino]-phenyl]-acetic acid or a base salt thereof other than the salt of formula I with codeine or a salt thereof.

3. A process according to claim 2, which comprises using at least an equimolar amount of codeine or of a salt thereof.

4. A pharmaceutical composition comprising the codeine salt of [2-[(2,6-dichlorophenyl)-amino]-phenyl]-acetic acid of formula I according to claim 1 together with customary pharmaceutical excipients.

5. The codeine salt of [2-[(2,6-dichlorophenyl)-amino]phenyl]-acetic acid of formula I according to claim 1 for use in a method for the therapeutic treatment of the human or animal body.

6. The use of the codeine salt of [2-[(2,6-dichlorophenyl)amino]-phenyl]-acetic acid of formula I according to claim 1 for the preparation of medicaments for the therapeutic treatment of the human or animal body.

## Revendications

1. Sel de codéine avec l'acide [2-[(2,6-dichlorophényl)amino]phényl]acétique de formule

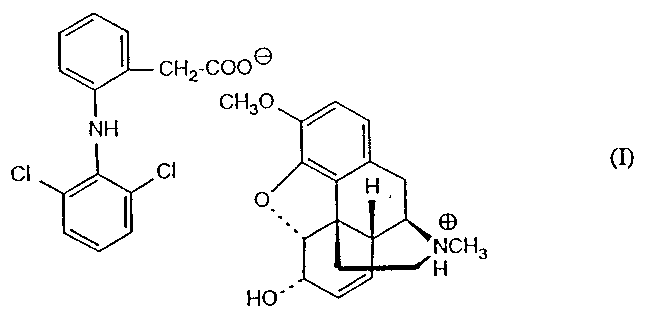

2. Procédé pour la préparation du sel de codéine avec l'acide [2-[(2,6-dichlorophényl)amino]phényl]acétique de formule I selon la revendication 1, caractérisé en ce que l'on fait réagir l'acide [2-[(2,6-dichlorophényl)amino]phényl]acétique ou un sel de celui-ci avec une base, différent du sel de formule I, avec la codéine ou un sel de celle-ci.

3. Procédé selon la revendication 2, caractérisé en ce que l'on utilise au moins une quantité équimolaire de codéine ou d'un sel de celle-ci.

4. Composition pharmaceutique contenant le sel de codéine avec l'acide [2-[(2,6-dichlorophényl)amino]phényl]acétique de formule I selon la revendication 1, en plus d'adjuvants pharmaceutiques usuels.

5. Sel de codéine avec l'acide [2-[(2,6-dichlorophényl)amino]phényl]acétique de formule I selon la revendication 1, pour son utilisations dans une méthode de traitement thérapeutique de l'organisme humain ou animal.

6. Utilisation du sel de codéine avec l'acide [2-[(2,6-dichlorophényl)amino]phényl]acétique de formule(I) selon la revendication 1, pour la fabrication de médicaments destinés au traitement thérapeutique de l'organisme humain ou animal.
